# EUROPEAN PATENT SPECIFICATION

(11) **EP 2 887 927 B1**
(45) Date of publication and mention of the grant of the patent: **28.03.2018**
(21) Application number: 13756040.5
(22) Date of filing: 22.08.2013
(51) Int. Cl.: A61K 9/70, A61K 9/14, A61K 9/20, A61K 9/48

(54) **EXTENDED RELEASE COMPOSITIONS OF AN AMINO-C2-C6-ALKYL NITRATE**
ZUSAMMENSETZUNG MIT VERLÄNGERTER FREISETZUNG EINES AMINO-C2-C6-ALKYLNITRATS
COMPOSITIONS À LIBÉRATION PROLONGÉE D'UN NITRATE AMINO-C2-C6-ALKYL

(30) Priority: 23.08.2012 EP 12181465
(43) Date of publication of application: 01.07.2015
(73) Proprietor: Cardiolynx AG, 4057 Basel (CH)
(72) Inventor: SARTOR, Dirk, 64688 Rimbach (DE); VANKAN, Pierre, CH-4125 Riehen (CH); STÜCKLER, Hubert, CH-4310 Rheinfelden (CH); SCHERHAG, Armin, CH-4143 Dornach (CH)
(74) Representative: Latscha Schöllhorn Partner AG
(86) International application number: PCT/EP2013/067452
(87) International publication number: WO 2014/029841

(56) References cited:
- WO-A1-2009/112167
- WO-A2-2008/064163

## Description

### Field of the invention

The present invention relates to extended release compositions of an amino-C₂-C₆-alkyl nitrate and of pharmaceutically acceptable salt thereof, and to fixed dose combinations with further pharmaceutically active drug substances, and methods of treatment of diseases using these compositions.

### Background of the invention

Nitrates have a long history of pharmaceutical use, causing vasodilatation and thereby increasing and improving blood flow. The main indications of nitrates are coronary heart disease, angina pectoris (chest pain), pulmonary hypertension, and congestive heart failure. There are several pharmaceutical compounds which are marketed and used in these indications. Examples are nitroglycerol, isosorbide dinitrate, and isosorbide mononitrate.

All of the presently marketed nitrates show a distinct disadvantage. Whereas initially the proposed dose efficiently provokes a vasodilatation, the patient will develop tolerance (nitrate tolerance) after a certain time, and the dose has to be increased continuously. At certain increased levels, toxic side effects will show up, preventing further safe use of the drug.

So far only one nitrate is known which does not provoke tolerance, AEN (2-aminoethyl nitrate). The corresponding drug product, Nilatil®, was marketed by Pharmacia AB, but later again withdrawn from the market. Whereas AEN shows an excellent pharmacological profile, in particular no development of nitrate tolerance, the short half-live of approx. 2 hours requires frequent dosing and causes high peak-to-trough ratios, which is not desirable since it compromises appropriate patient compliance.

AEN was protected under SE 168 308, its use under US 3,065,136.

Pharmaceutical formulations useful for sustained drug release became an important part of medication in terms of improved treatment effect, reduction of side effects and patients' convenience. Extended release of an active drug provides many therapeutic advantages, the most important of which is that the drug blood levels are maintained for a long time with minimal fluctuation. The problem with each dose of a standard immediate release drug is that the concentration of drug available to the body immediately peaks and then declines rapidly. When the drug concentration reaches very high levels it contributes to adverse side effects, while when it remains at lower levels it does not provide any therapeutic benefit. Thus, it is desirable to release drugs at a constant rate, thereby maintaining drug concentration within the therapeutic range and eliminating the need for frequent dosages. Other advantages of extended release devices include: delivery to the required site, reduced risk of overdose with side effects, and economic advantage by virtue of a more efficient dosage.

There are several designations found in literature to describe formulations that do not release the active drug substance immediately following oral administration. The term used in a document by the Center for Drug Evaluation and Research (Guidance for Industry, Extended Release Oral Dosage Forms: Development, Evaluation, and Application of In Vitro/In Vivo Correlations; Food and Drug Administration, Center for Drug Evaluation and Research (CDER), September 1997) is "extended release dosage form". Other designations are "sustained release formulation", "controlled release formulation" or "prolonged action formulation".

Modified-release preparations can be administered orally in single or multiple-unit dosage forms. Single-unit formulations contain the active ingredient within the single tablet or capsule, whereas multiple-unit dosage forms comprise a number of discrete particles that are combined into one dosage unit, so as to form a multiple-unit system. Multiparticulates may not only be presented as capsule formulations, but as well as tablets.

In matrix devices, the active agent appears as a dispersion within the polymer matrix and is typically formed by simple compression or wet granulation followed by compression of a polymer/drug mixture. Matrix formulations are probably the most common devices used for controlling the release of drugs.

Among the various technologies used to control the systemic delivery of drugs, osmotic drug delivery is one of the most interesting and widely applicable. Osmotic drug delivery uses the osmotic pressure of drugs or other solutes for controlled delivery of drugs. Osmotic drug delivery systems offer distinct advantages which have contributed to the popularity of these systems (Verma RK et al., Drug Dev Ind Pharm 2000; 26:695-708).

The best known systems are those originally developed by ALZA Corp. under the trade name OROS®. Specifically, ODDS systems were as well used for nitrates, e.g. for isosorbide mononitrate (Verma RK et al., Intl J of Pharmaceutics 2003; 263:9-24).

Prolonged gastric retention systems have been studied to improve the *in vivo* behavior of extended-release dosage forms. The basic concept is that, being exposed to a more or less constant environment in the stomach, the *in vivo* release of the dosage forms will be more controllable. These forms are based on a range of totally different concepts, recently reviewed by Streubel A et al., Expert Opin Drug Deliv 2006; 3:217-233.

Skin is the largest organ of the body and forms a protective barrier to the entry of foreign molecules. It is especially impermeable to water-soluble compounds, even in small quantities. However, moderately lipophilic drugs have been delivered through the skin for several years. Since the early 1970s, there has been a significant effort to develop commercially viable transdermal formulations. Transdermal patch formulations of nitroglycerin, isosorbide dinitrate, clonidine, fentanyl, piroxicam, ketoprofen, nicotine, scopolamine, estradiol, norethisterone, levonorgestrel, and testosterone have been developed and commercialized in the cardiovascular, pain management, smoking cessation, hormone replacement therapy, and motion sickness markets.

Transdermal drug delivery circumvents first pass metabolism associated with the oral delivery of drugs, therefore delivery of certain compounds from the skin can require significantly smaller amounts of drugs than the corresponding oral dosage, potentially reducing dosage-related side-effects.

Traditionally, low dose drugs with first pass metabolism problem after oral administration have been the ideal candidates for transdermal delivery. Increasing skin permeation rates for drugs therefore is a challenge that must be overcome for achieving target flux rates for therapeutic efficacy. The research over the past two decades has identified hundreds of novel technologies for overcoming skin barrier properties. Almost all of these technologies breach the top impervious barrier, stratum corneum, using chemicals, sound, light, heat, micro needles and electrical current.

### Summary of the invention

The present invention relates to extended release compositions of an amino-C₂-C₆-alkyl nitrate, in particular of 2-aminoethyl nitrate, and of pharmaceutically acceptable salts thereof.

The invention further relates to a fixed dose combination of an amino-C₂-C₆-alkyl nitrate, in particular of 2-aminoethyl nitrate, and of pharmaceutically acceptable salts thereof with further pharmaceutically active drug substances.

The invention further relates to a method of treatment of diseases using an extended release composition of an amino-C₂-C₆-alkyl nitrate, alone or in combination with other pharmaceutically active drug substances.

### Brief description of the figures

**Figure 1****:** Dissolution profiles of five different formulations of AEN tosylate (Ph. Eur.; paddle, pH 6.8, 37°C) based on ethylcellulose coating.
   Horizontal axis: Time in hours. Vertical axis: % of AEN tosylate dissolved from formulation 1, 2, 3, 4 and 5, respectively. The formulations are described in the experimental part.
**Figure 2****:** Dissolution profiles of two formulations of AEN tosylate (Ph. Eur.; paddle, pH 6.8, 37°C) with a double layer of ethylcellulose and Kollicoat SR.
   Horizontal axis: Time in hours. Vertical axis: % of AEN tosylate dissolved from formulation 6 and 7, respectively. The formulations are described in the experimental part.
**Figure 3****:** Plasma concentration of AEN tosylate in beagle dogs dosed with formulation 6 applied at 100 mg/kg.
   Horizontal axis: Time in hours. Vertical axis: Logarithmic plot of plasma concentration in ng/ml. Data from 3 different dogs (dog 1, dog 2 and dog 3).
**Figure 4****:** Plasma concentration of AEN tosylate in beagle dog dosed with formulation 7 applied as 10 mg/animal.
   Horizontal axis: Time in hours. Vertical axis: Logarithmic plot of plasma concentration in ng/ml.
**Figure 5****:** Pharmacological activity of AEN tosylate, valsartan and AEN tosylate/valsartan combination on blood pressure in L-NAME treated SHR rats.
   Column 1: AEN tosylate (0.5 mg/kg/h), column 2: valsartan (single dose 5 mg/kg), column 3: AEN tosylate (0.5 mg/kg/h) plus valsartan (single daily dose 5 mg/kg).
   Hatched bars: blood pressure lowering versus control after 1 week, black solid bars after 2 weeks. Vertical axis: reduction in blood pressure in mm Hg

### Detailed description of the invention

The present invention relates to extended release compositions of an amino-C₂-C₆-alkyl nitrate in form of the free base or of a pharmaceutically acceptable salt.

C₂-C₆-alkyl is an alkyl group of consisting of 2 to 6 carbon atoms, in particular ethyl, propyl, butyl, pentyl and hexyl. The alkyl group may be linear, as in n-propyl, n-butyl, n-pentyl and n-hexyl, or branched, as for example in iso-propyl, iso-butyl, sec-butyl, tert-butyl, iso-pentyl, 1-or 2-methylbutyl, 1-ethylpropyl, 1,2-dimethylpropyl, tert-pentyl, and corresponding branched hexyl groups, e.g. iso-hexyl and 1, 2, and 3-methyl-pentyl.

A C₂-C₆-alkyl nitrate is sometimes also called C₂-C₆-alkanol nitrate, indicating more clearly that a nitrate is an ester of nitric acid with the corresponding alkanol, or also a nitro-oxyalkane. In the context of the present invention it is understood that in an alkyl nitrate the nitrate function is covalently bonded to the alkyl residue by an oxygen atom.

In amino-C₂-C₆-alkyl nitrate, the amino group and/or the nitrate function may be in a primary, secondary or tertiary position, if possible at all. Preferably, the amino group is not bound to the same carbon atom as the nitrate function. More preferably, both amino group and nitrate function are in a primary position, for example as in 2-aminoethyl nitrate, 3-amino-propyl nitrate, 4-aminobutyl nitrate, 5-aminopentyl nitrate, 6-aminohexyl nitrate, 3-amino-2-methylpropyl nitrate, and 3-amino-2,2-dimethylpropyl nitrate. However, other substitution patterns are also considered, for example as in 2-amino-1-methylethyl nitrate, 3-amino-1-methylpropyl nitrate, 2-amino-1,1-dimethylethyl nitrate, 2-aminopropyl nitrate, 2-aminobutyl nitrate, and 2-amino-3-methylbutyl nitrate.

Preferred amino-C₂-C₆-alkyl nitrates are 4-aminobutyl nitrate, 3-aminopropyl nitrate, 2-amino-1-methylethyl nitrate, and 2-aminoethyl nitrate, in particolar 4-aminobutyl nitrate and 2-aminoethyl nitrate. Most preferred is 2-aminoethyl nitrate (AEN), also known under the name itramine.

Pharmaceutically acceptable salts of C₂-C₆-alkyl nitrates are acid addition salts of pharmaceutically acceptable non-toxic inorganic and organic acids. Preferred pharmaceutically acceptable salts are acetate, benzoate, besylate (benzenesulfonate), bromide, chloride, camphorsulfonate, chlortheophyllinate, citrate, ethenedisulfonate, fumarate, gluconate, glutamate, hippurate, 2-hydroxyethanesulfonate, 2-hydroxy-2-phenylacetate, iodide, lactate, laurylsulfate, malate, maleate, mesylate (methanesulfonate), methylsulfate, napsylate (2-naphthalenesulfonate), nitrate, octadecanoate, oxalate, pamoate, phosphate, polygalacturonate, succinate, sulfate, tartrate, and tosylate (p-toluenesulfonate).

Most preferred pharmaceutically acceptable salts are sulfate, phosphate, acetate and tosylate, in particular tosylate.

Accordingly, it is an object of the present invention to provide an extended release composition of an amino-C₂-C₆-alkyl nitrate and pharmaceutically acceptable salts thereof allowing once or twice daily administration of the drug product.

The term "extended release" as used herein in relation to the composition according to the invention or a coating or coating material or used in any other context means release which is not immediate release, but release over a pre-defined, longer time period of up to 24 hours, such as 4-24 hours, e.g. 6-24 hours, preferably 12-24 hours. Release characteristics and release time are measured according to standard methods, e.g. those of Ph. Eur., in aqueous solution with a paddle at pH 6.8 and 37°C.

The extended-release characteristics for the release of amino-C₂-C₆-alkyl nitrate and pharmaceutically acceptable salts thereof may be varied by modifying the composition of each formulation component, including modifying any of the excipients and coatings or also transdermal patch layers which may be present. In particular the release of the active ingredient may be controlled by changing the composition and/or the amount of the extended-release coating, if such a coating is present. If more than one extended-release component is present, the coating or matrix former for each of these components may be the same or different. Similarly, when extended-release is governed by an extended-release matrix material, release of the active ingredient may be controlled by the choice and amount of extended-release matrix material utilized. However, the use of ion exchange resin as the extended-release matrix material is excluded from the present invention.

When the extended-release component comprises a modified release matrix material, any suitable extended-release matrix material or suitable combination of extended-release matrix materials except ion exchange resin may be used. Such materials are known to those skilled in the art. The term "extended-release matrix material" as used herein includes hydrophilic polymers, hydrophobic polymers and mixtures thereof which are capable of modifying the release of an active ingredient dispersed therein *in vitro* and *in vivo.*

In a preferred embodiment, the extended-release composition according to the present invention will provide more or less constant plasma levels of amino-C₂-C₆-alkyl nitrate and pharmaceutically acceptable salts thereof over 6 hours, 12 hours, more preferably over 24 hours. In case of a fixed-dose combination product, such a plasma profile produced from the administration of a single dosage unit is especially advantageous without the need for administration of two dosage units.

One of the objects of this invention is to provide an extended release oral dosage form.

For oral dosage forms, any coating material which modifies the release of the active ingredient in the desired manner may be used. In particular, coating materials suitable for use in the practice of the invention include but are not limited to polymer coating materials, such as cellulose acetate phthalate, cellulose acetate trimellitate, hydroxy propyl methylcellulose phthalate, polyvinyl acetate phthalate, ammonio methacrylate copolymers such as those sold under the trademark Eudragit® RS and RL, polyacrylic acid and polyacrylate and methacrylate copolymers such as those sold under the trademark Eudragit® S and L, polyvinyl acetaldiethylamino acetate, hydroxypropyl methylcellulose acetate succinate, shellac; hydrogels and gel-forming materials, such as carboxyvinyl polymers, sodium alginate, sodium carmellose, calcium carmellose, sodium carboxymethyl starch, polyvinyl alcohol, hydroxyethyl cellulose, methyl cellulose, gelatin, starch, and cellulose based cross-linked polymers in which the degree of crosslinking is low so as to facilitate adsorption of water and expansion of the polymer matrix, hydoxypropyl cellulose, hydroxypropyl methylcellulose, polyvinyl pyrrolidone, crosslinked starch, microcrystalline cellulose, chitin, aminoacryl-methacrylate copolymer (Eudragit® RS-PM), pullulan, collagen, casein, agar, gum arabic, and sodium carboxymethyl cellulose.

In a particular embodiment of the invention, non-ionic matrix material is used.

As will be appreciated by the person skilled in the art, excipients such as plasticisers, lubricants, solvents and the like may be added to the coating. Suitable plasticisers include, for example, acetylated monoglycerides, butyl phthalyl butyl glycolate, dibutyl tartrate, diethyl phthalate, dimethyl phthalate, ethyl phthalyl ethyl glycolate, glycerol, propylene glycol, triacetin, citrate, tripropionin, diacetin, dibutyl phthalate, acetyl monoglyceride, polyethylene glycols, castor oil, triethyl citrate, polyhydric alcohols, acetyl triethyl citrate, dibenzyl phthalate, dihexyl phthalate, butyl octyl phthalate, di-isononyl phthalate, butyl octyl phthalate, dioctyl azelate, epoxidized tallate, tri-isoctyl trimellitate, diethylhexyl phthalate, di-n-octyl phthalate, di-isooctyl phthalate, di-isodecyl phthalate, di-n-undecyl phthalate, di-n-tridecyl phthalate, tri-2-ethylhexyl trimellitate, di-2-ethylhexyl adipate, di-2-ethylhexyl sebacate, di-2-ethylhexyl azelate, and dibutyl sebacate.

For oral dosage forms, extended release of the active ingredient in the desired manner may be obtained by embedding the drug in a matrix. In matrix devices, the active agent appears as a dispersion within the polymer matrix and is typically formed by the simple compression of a polymer/drug mixture, through its dissolution in a common solvent or melt granulation. Matrix formulations are particularly preferred for controlling the release of the active ingredient of the present invention, since they are relatively easy to manufacture compared to other devices.

Among the many oral dosage forms that can be used for extended drug release, matrix tablets, as obtained by the direct compression of a polymer mixture, are preferred. Matrix materials considered are biocompatible natural polymers, e.g. HPMC, HEMC, EHEC, HMHEC, CMHEC, methylcellulose, guar, pectin, agar, algin, gellan gum, xanthan gum, acacia, starch and modified starches, carrageenans, amylose starch, and the like. Ion exchange resins are excluded from the scope of the invention.

Particular swellable hydrophilic polymers considered as matrix materials are poly-(hydroxyalkanol methacrylate) (MW 5 kD to 5,000 kD), polyvinylpyrrolidone (MW 10 kD to 360 kD), anionic and cationic hydrogels, polyvinyl alcohol having a low acetate residual, a swellable mixture of agar and carboxymethyl cellulose, copolymers of maleic anhydride and styrene, ethylene, propylene or isobutylene, pectin (MW 30 kD to 300 kD), polysaccharides such as agar, acacia, karaya, tragacanth, algins and guar, polyacrylamides, Polyox® polyethylene oxides (MW 100 kD to 5,000 kD), AquaKeep® acrylate polymers, diesters of polyglucan, crosslinked polyvinyl alcohol and poly N-vinyl-2-pyrrolidone, and sodium starch glucolate (e.g. Explotab®; Edward Mandell Co. Ltd.).

Further particular hydrophilic polymers considered are polysaccharides, methyl cellulose, sodium or calcium carboxymethyl cellulose, hydroxypropyl methyl cellulose, hydroxypropyl cellulose, hydroxyethyl cellulose, nitro cellulose, carboxymethyl cellulose, cellulose ethers, polyethylene oxides (e.g. Polyox®, Union Carbide), methyl ethyl cellulose, ethyl hydroxyl-ethylcellulose, cellulose acetate, cellulose butyrate, cellulose propionate, gelatin, collagen, starch, maltodextrin, pullulan, polyvinyl pyrrolidone, polyvinyl alcohol, polyvinyl acetate, glycerol fatty acid esters, polyacrylamide, polyacrylic acid, copolymers of methacrylic acid or methacrylic acid (e.g. Eudragit®), other acrylic acid derivatives, sorbitan esters, natural gums, lecithins, pectin, alginates, ammonium alginate, sodium, calcium and/or potassium alginates, propylene glycol alginate, agar, and gums such as arabic, karaya, locust bean, tragacanth, carrageens, guar, xanthan, scleroglucan and mixtures and blends thereof.

Preferred modified release matrix materials suitable for the practice of the present invention are microcrystalline cellulose, sodium carboxymethylcellulose, hydoxyalkyl-celluloses such as hydroxypropylmethylcellulose and hydroxypropylcellulose, polyethylene oxide, alkylcelluloses such as methylcellulose and ethylcellulose, polyethylene glycol, polyvinylpyrrolidone, cellulose acteate, cellulose acetate butyrate, cellulose acteate phthalate, cellulose acteate trimellitate, polyvinylacetate phthalate, polyalkylmethacrylates, polyvinyl acetate, and mixture thereof.

Another object is to provide a multiparticulate extended release composition.

The term "particulate" as used herein refers to a state of matter which is characterized by the presence of discrete particles, pellets, beads, granules, or small tablets ("mini-tablets") irrespective of their shape or morphology.

The term "multiparticulate" as used herein means a plurality of discrete, or aggregated, particles, pellets, beads, granules, small tablets or mixture thereof irrespective of their shape or morphology. The term "multiparticulate" includes every subunit of a size smaller than 5 mm, e.g. pellets, granules, sugar seeds (non-pareil), minitablets, powders, and crystals, with drugs being entrapped in or layered around cores.

Although similar *in vitro* drug release profiles can be obtained with single-unit and multiple-unit dosage forms, the latter offer several advantages over single-unit systems such as non-disintegrating tablets or non-disintegrating capsules, and represent a preferred embodiment of the invention.

Multiparticulates according to the invention are filled into capsule or compressed into tablets. Tablets allow to insert a breaking score, making it possible to sub-divide the dose, and still maintaining the extended-release characteristics of the multiparticulate formulation. Multiparticulates provide many advantages over single-unit systems because of their small size. They are less dependable upon gastric emptying, resulting in less inter- and intra-subject variability in gastrointestinal transit time. They are also better distributed and less likely to cause local problems. Other advantages include adjustment of the strength of a dosage unit by changing the number of multiparticulates in the unit, administration of incompatible drugs in a single dosage unit by separating them in different multiparticulates, and combination of multiparticulates with different drug release rates to obtain the desired overall release profile. In multiple unit systems, the total drug dose is divided over multiparticulates that make up that system. Failure of a few units may not be as consequential as failure of a single-unit system, where a failure may lead to dose-dumping of the drug.

Still another object of the present invention is to provide a single-unit extended release tablet-, film coated tablet- or hard capsule formulation of an amino-C₂-C₆-alkyl nitrate and pharmaceutically acceptable salts thereof. The term "hard capsules" includes any type of hard capsule made from gelatin or a different material, e.g. hypromellose and gellan gum (Vcaps™) or pullulan and carrageenan (NPcaps™).

Another object of the present invention is to provide an osmotically controlled oral dosage form of an amino-C₂-C₆-alkyl nitrate and pharmaceutically acceptable salts thereof. Drug delivery from an osmotic drug delivery system is not influenced by the different physiological factors within the gut. Osmotic drug delivery systems (ODDS), apart from maintaining plasma concentration within therapeutic range, also prevent sudden increase in plasma concentration that may produce side effects and sharp decrease in plasma concentrations that may reduce the efficacy of the drug. Considered are single-layer core osmotic pumps using conventional tablets as a core. Elementary osmotic pump (EOP) and the controlled porosity (OP) are two different embodiments of this technology. Further preferred are dosage forms based on the so-called "push-pull system" using a bi-layer kernel (multi-layer core osmotic pumps).

A further object of the present invention is to provide a transdermal drug delivery system of an amino-C₂-C₆-alkyl nitrate and pharmaceutically acceptable salts thereof, further comprising another active ingredient in a fixed-dose combination as described below, releasing the active drugs continuously over a time window of approximately 24 hours or more, e.g. up to 7 days.

The skin permeation rate of amino-C₂-C₆-alkyl nitrates are positively influenced by chemical enhancers, for example cosolvents such as ethanol, isopropanol, glycerol, polyethylene glycol, propylene glycol, pyrrolidones, dimethylsulfoxide, laurocapram (1-dodecylazepan-2-one) and the like, surfactants, fatty acid esters such as polyethylene glycol monolaurate, and terpenes such as menthol. Preferred dermal penetration enhancers are laurocapram and laurocapram derivatives, and oleic acid and its esters, such as methyl, ethyl, propyl, isopropyl, butyl, vinyl and glyceryl esters, dodecyl (N,N-dimethylamino)acetate and dodecyl (N,N-dimethylamino)propionate, and 2-n-nonyl-1-3-dioxolane. Most preferred dermal penetration enhancers are oleic acid and its esters, dodecyl (N,N-dimethylamino)-acetate and -propionate, and 2-n-nonyl-1-3-dioxolane. The penetration enhancers facilitate the delivery of drugs through the skin by temporarily altering the top skin barrier layer.

A variety of transdermal technologies, including reservoir patches, matrix patches, poration devices and iontophoretic devices are considered. Preferred are drug-in-adhesive and reservoir patches. The drug-in-adhesive patches combine adhesive and the drug in a single layer making it less costly to manufacture. Such patches may be used for several days, e.g. up to 7 days, are light weight and thin, and can be rather small and translucent.

Poration technologies and iontophoretic delivery are not of relevance for extended-release delivery of amino-C₂-C₆-alkyl nitrates and pharmaceutically acceptable salts thereof, but are of interest for fixed-dose combination therapy with further drugs which show a very low permeation through human skin.

Drug-in-adhesive systems incorporate amino-C₂-C₆-alkyl nitrates into a carrier such as a polymeric matrix and/or a pressure-sensitive adhesive, such as silicone adhesive, silicone rubber, acrylic adhesive, polyethylene, polyisobutylene, polyvinyl chloride, nylon, or the like formulation. The pressure-sensitive adhesive must adhere effectively to the skin and permit migration of the medicament from the carrier through the skin and into the bloodstream of the patient. The amino-C₂-C₆-alkyl nitrate is directly incorporated into the adhesive in one single layer, or alternatively dissolved in the polymeric matrix until its saturation concentration is reached. Any additional drug remains dispersed within the matrix. When drug migrates through the skin and thereby is removed from the surface of the matrix, more of the drug diffuses out of the interior in response to the decreased concentration at the surface. The release rate is therefore not constant over time, but instead gradually decreases as the drug concentration decreases.

Reservoir patches contain a reservoir or a pocket which holds the amino-C₂-C₆-alkyl nitrates, encapsulated in a gel. A protective seal covers the contents of the patch. A permeable film allows the nitrates to flow through at a controlled rate.

In order to modify the rate of delivery from the transdermal device, a specific single-polymer matrix or a blend of soluble (miscible) polymers is considered. Polymers considered are those listed above for oral drug forms.

The drug-in-adhesive patches are manufactured by following sequence: Appropriate amounts of adhesives are dissolved in a solvent in a vessel. The amino-C₂-C₆-alkyl nitrate or a pharmaceutically acceptable salt thereof is added and dissolved/dispersed in a polymer mixture, and optional co-solvents and enhancers are added. The solution is coated onto a protective release liner at a controlled specified thickness. Then the coated product is passed through an oven to drive off volatile solvents. The dried product on the release liner is combined with the backing material and wound into rolls for storage. The roll of film is then cut at the desired size and packaged.

Another object of the present invention is to provide a fixed-dose combination of an amino-C₂-C₆-alkyl nitrate and pharmaceutically acceptable salts thereof and further pharmacologically active drug substances, whereby the amino-C₂-C₆-alkyl nitrate and pharmaceutically acceptable salts thereof are provided in extended release form, and the further drug substances will be preferably in immediate release form.

Any pharmaceutically active compound for which a combination therapy with amino-C₂-C₆-alkyl nitrate is useful may be combined in a fixed-dose combination according to the invention. Compounds falling into this category are:
Sartans (angiotensin receptor blockers, ARB's), angiotensin enzyme inhibitors (ACE-I), renin antagonists and other classes of antihypertensive drugs (e.g. beta blocker, calcium channel blockers (CCB's) for the treatment of arterial hypertension;
endothelin antagonists such as bosentan, mazisentan and others for the treatment of pulmonary hypertension;
statins and other drugs indicated for the treatment of dyslipidaemias, especially LDL cholesterol-lowering drugs;
biguanides such as metformin, thiazolidinediones (glitazones) such as pioglitazone or aleglitazar, sulfonureas such as glibenclamide or glimepiride, SGLT2 antagonists such as dapagliflozin, canagliflozin, ipragliflozin, tofogliflozin and empagliflozin, dipeptidyl peptidase-(DPP)-4 inhibitors such as sitagliptin, vildagliptin, saxagliptin and linagliptin, and any other oral drug indicated for the treatment and prevention of diabetes and/or diabetic complications;
antithrombotics, antiplatelets, anticoagulants and other drug indicated for the treatment or prevention of thrombosis or other coagulopathies;
antianginal drugs such as beta blockers, calcium channel antagonists (CCB's), ranolazine, ivabradine and others for the treatment of stable chronic angina pectoris;
vasodilators such hydralazine, cilostazol and other phospodiesterase III inhibitors (PDE III) indicated for the treatment of peripheral artery disease or hypertension or acute or chronic heart failure (CHF) or vascular diseases; and
aldosterone antagonists such as spironolactone, eplerenone and aldosterone synthase inhibitors (ASI's) indicated for the treatment of chronic heart failure (CHF) or chronic kidney diseases or pulmonary hypertension.

It is to be understood that this list is made only for the purpose of describing or providing various pharmaceutically active compounds which may be used in combination with amino-C₂-C₆-alkyl nitrate and pharmaceutically acceptable salts thereof. The list is not intended to limit the scope of the present invention. All such pharmaceutically active compounds for which it is clinically reasonable to be used in a combination therapy are thus within the scope of the present invention.

Preferred pharmaceutically active compounds for a fixed-dose combination with amino-C₂-C₆-alkyl nitrate and pharmaceutically acceptable salts thereof are valsartan, azilsartan, mazisentan, cilostazol, pioglitazone, sitagliptin and linagliptin, in particular valsartan, cilostazol and pioglitazone.

Amino-C₂-C₆-alkyl nitrate and pharmaceutically acceptable salts thereof, calculated as free base, are preferably present in a composition in an amount of from 0.01 to 100 mg, preferably in the amount of from 0.1 to 50 mg.

In case of fixed-dose combination products, the ratio of amino-C₂-C₆-alkyl nitrate and pharmaceutically acceptable salts thereof to the second pharmaceutically active drug substance may vary in a wide range. Additionally, for a given qualitative combination of amino-C₂-C₆-alkyl nitrate and pharmaceutically acceptable salts thereof and the second drug substance, different ratios may be selected to adjust for the individual needs of the patient.

Still another object of the present invention is to provide a fixed-dose combination, whereby multiple units of an amino-C₂-C₆-alkyl nitrate and pharmaceutically acceptable salts thereof and multiple units of the additional drug substance are mixed and filled into hard capsules. In this case, the further drug may be present in form of a conventional granulate known to the skilled person in the art.

Another object of the present invention is to provide a fixed-dose combination, whereby multiple units of an amino-C₂-C₆-alkyl nitrate and pharmaceutically acceptable salts thereof and a powder or granulate containing the additional drug substance are mixed and compressed into tablets. These tablets may be further film-coated to provide advantages known to the skilled person in the art, for instance to protect from an unpleasant taste, or to provide a distinctive color.

Still a further object of the present invention is to provide a fixed-dose combination, whereby a single unit formulation of an amino-C₂-C₆-alkyl nitrate and pharmaceutically acceptable salts thereof with extended release characteristics is press-coated with a powder or granulate containing the additional drug substance. The resulting tablet may again be film-coated.

Still a further object of the present invention is to provide a fixed-dose combination transdermal drug delivery system of an amino-C₂-C₆-alkyl nitrate and pharmaceutically acceptable salts thereof, and further pharmacologically active compounds, releasing both active drugs continuously over a time window of approximately 12-24 hours or more.

Still a further object of the invention is to provide a fixed-dose combination, whereby amino-C₂-C₆-alkyl nitrate and pharmaceutically acceptable salts thereof are provided in one dosage form and the additional drug substance in another dosage form, and both dosage forms are provided in a kit with instruction to use both dosage forms together. In particular, the amino-C₂-C₆-alkyl nitrate and pharmaceutically acceptable salts thereof may be in a dosage form as described above, and the additional drug substance in a standard dosage form particularly adapted to said additional drug substance. Both the amino-C₂-C₆-alkyl nitrate and the additional drug substance may be provided in suitable oral formulations, or the amino-C₂-C₆-alkyl nitrate in a transdermal drug delivery system and the additional drug substance in a suitable oral formulation,

The invention further relates to a method of treatment of diseases using an extended release composition of an amino-C₂-C₆-alkyl nitrate, alone or in combination with other pharmaceutically active drug substances. Preferred amino-C₂-C₆-alkyl nitrates are those indicated above, in particular 2-aminoethyl nitrate (AEN), also known under the name itramine.

In particular the invention relates to a method of treatment of arterial hypertension, Raynaud's disease, morbus Menière, or argininosuccinic aciduria (ASA), in particular argininosuccinic aciduria, comprising administering a therapeutically effective amount of an extended release composition of an amino-C₂-C₆-alkyl nitrate, in particular of 2-aminoethyl nitrate.

Furthermore the invention relates to a methods of treatment of particular diseases using an extended release composition of an amino-C₂-C₆-alkyl nitrate in combination with other pharmaceutically active drug substances known to treat such disease, thereby increasing the efficiency of the treatment and allowing to reduce the dose of the other pharmaceutically active drug substances when applied in combination with an amino-C₂-C₆-alkyl nitrate.

In one embodiment the invention relates to a method of treatment of arterial hypertension comprising administering a therapeutically effective amount of an extended release composition of an amino-C₂-C₆-alkyl nitrate, in particular of 2-aminoethyl nitrate, in combination with a sartan, angiotensin enzyme inhibitor, renin antagonist or other antihypertensive drug.

In another embodiment the invention relates to a method of treatment of pulmonary hypertension comprising administering a therapeutically effective amount of an extended release composition of an amino-C₂-C₆-alkyl nitrate, in particular of 2-aminoethyl nitrate, in combination with an endothelin antagonist.

In another embodiment the invention relates to a method of treatment of dyslipidaemia comprising administering a therapeutically effective amount of an extended release composition of an amino-C₂-C₆-alkyl nitrate, in particular of 2-aminoethyl nitrate, in combination with a statin or other LDL cholesterol-lowering drug.

In another embodiment the invention relates to a method of treatment of diabetes or diabetic complications comprising administering a therapeutically effective amount of an extended release composition of an amino-C₂-C₆-alkyl nitrate, in particular of 2-aminoethyl nitrate, in combination with a biguanide, thiazolidinedione, sulfonurea, SGLT2 antagonist, or dipeptidyl peptidase-4 inhibitor.

In another embodiment the invention relates to a method of treatment or prevention of thrombosis or other coagulopathies comprising administering a therapeutically effective amount of an extended release composition of an amino-C₂-C₆-alkyl nitrate, in particular of 2-aminoethyl nitrate, in combination with an antithrombotic, antiplatelet or anticoagulant drug.

In another embodiment the invention relates to a method of treatment of stable chronic angina pectoris comprising administering a therapeutically effective amount of an extended release composition of an amino-C₂-C₆-alkyl nitrate, in particular of 2-aminoethyl nitrate, in combination with a beta blocker, calcium channel antagonist, ranolazine, or ivabradine.

In another embodiment the invention relates to a method of treatment of peripheral artery disease, hypertension, acute or chronic heart failure or vascular disease comprising administering a therapeutically effective amount of an extended release composition of an amino-C₂-C₆-alkyl nitrate, in particular of 2-aminoethyl nitrate, in combination with a hydralazine, cilostazol or phospodiesterase III inhibitor.

In another embodiment the invention relates to a method of treatment of chronic heart failure, chronic kidney disease or pulmonary hypertension comprising administering a therapeutically effective amount of an extended release composition of an amino-C₂-C₆-alkyl nitrate, in particular of 2-aminoethyl nitrate, in combination with a spironolactone, eplerenone or aldosterone synthase inhibitor.

The preferred dose of 2-aminoethyl nitrate, applied in an extend release composition as monotherapy or in combination therapy with other active ingredients, is 6 to 50 mg daily for a human patient of around 70 kg, preferably 10-25 mg daily.

### Examples

### Example 1: Multiparticulates based on sugar spheres prepared by layering technique

### Example 1a: 2-Aminoethylnitrate (AEN) tosylate

### Example 1b: 4-Aminobutylnitrate tosylate.

| | Example 1a | | Example 1b | |
|---|---|---|---|---|
| | mg | % (dry matter) | mg | % (dry matter) |
| AEN tosylate | 15.75 | 7.50 | --- | --- |
| 4-Aminobutylnitrate tosylate | --- | --- | 18.28 | 8.60 |
| Polyvinylpyrrolidone K-30 | 12.25 | 5.83 | 12.25 | 5.76 |
| *Sub-total (solids)* | *28. 00* | | *30.53* | |
| Water, purified, q.s. | 150.00 | | 150.00 | |
| *Sub-total (solution*/*suspension)* | *178.00* | | *180.53* | |
| Sugar spheres (45-50 mesh) (300-355 µm) | 150.00 | 71.43 | 150.00 | 70.58 |
| *Sub-total (layered sugar spheres)* | *178.00* | | *180.53* | |
| Opadry® II (Sealcoat) (dry matter) | 10.00 | 4.76 | 10.00 | 4.71 |
| *Sub-total (layered sealcoated sugar spheres)* | *188.00* | | *190.53* | |
| Aqueous ethylcellulose dispersion (Surelease®) (drymatter)*) | 19.00 | 9.05 | 19.00 | 8.94 |
| Opadry® II (top coat) | 3.00 | 1.43 | 3.00 | 1.41 |
| Total | 210.00 | 100.00 | 212.53 | 100.00 |

| | | | | |
|---|---|---|---|---|
| *) includes film-forming polymer, plasticizer and stabilizer | | | | |

AEN tosylate or 4-aminobutylnitrate tosylate, respectively, is suspended in an aqueous solution of polyvinylpyrrolidone K-30 (approx. 15% solids). Sugar spheres of appropriate size are layered with the AEN tosylate - polyvinylpyrrolidone suspension or 4-aminobutylnitrate tosylate - polyvinylpyrrolidone suspension, respectively, in a fluid bed equipment, and the layered spheres are thoroughly dried. A seal coat of Opadry® is applied in a fluid bed equipment, followed by an extended-release layer of ethylcellulose (in form of a Surelease® dispersion). A final top coat of Opadry® is then applied. The coated multiparticulates are cured at 60°C for 12 hours, to ensure that polymer particles coalesce to form a smooth membrane on extended release multiparticulates. A quantity of approx. 210 mg of the final beads are filled into hard capsules of size "2".

### Example 2: (AEN) multiparticulates based on microcrystalline cellulose pellets prepared by layering technique

| | mg | %(dry matter) |
|---|---|---|
| AEN tosylate | 26.24 | 10.43 |
| Hypromellose (substitution type 2910, PHARMACOAT® 603) | 13.51 | 5.37 |
| Polyethylene glycol 6000 | 0.25 | 0.10 |
| *Sub-total (solids)* | *40.00* | |
| Alcohol USP / ethanol (96%) EP | 60.00 | |
| Water, purified, q.s. | 100.00 | |
| *Sub-total (solution*/*suspension)* | *200.00* | |
| Microcrystalline cellulose spheres (Cellets® 100) (65-150 mesh) (100-200 µm) | 200.00 | 79.52 |
| *Sub-total (layered cellulose spheres)* | *240.00* | |
| Hydroxypropylcellulose (Klucel®) (Sealcoat) (dry matter) | 2.50 | 0.99 |
| Dibutyl sebacate | 0.25 | 0.10 |
| Water, purified, q.s. | 20.00 | |
| *Sub-total (layered, sealcoated cellulose spheres)* | *242.50* | |
| EUDRAGIT® RL 30 D ("Ammonio Methacrylate Copolymer Dispersion, Type A" NF) (1 part) (30% dry matter) | 1.20 | 0.14 |
| EUDRAGIT® RS 30 D ("Ammonio Methacrylate Copolymer Dispersion, Type B" NF) (9 parts) (30% dry matter) | 10.60 | 1.26 |
| Triethyl citrate | 0.70 | 0.28 |
| Talc | 1.76 | 0.70 |
| Water | 15.75 | |
| *Sub-total (solution*/*suspension)* | *30.01* | |
| *Sub-total (layered, sealcoated, extended release coated cellulose spheres)* | *248.50* | |
| Opadry® II (top coat) | 3.00 | 1.19 |
| Total | 251.50 | 100.0 |

AEN tosylate is suspended in an aqueous-ethanolic solution of hypromellose and polyethylene glycol (approx. 20% solids). Microcrystalline cellulose spheres of appropriate size are layered with the AEN tosylate - hypromellose - polyethylene glycol suspension in a fluid bed equipment, and the layered spheres are thoroughly dried. A seal coat of hydroxypropylcellulose, plasticized with dibutyl sebacate, is applied in a fluid bed equipment, followed by an extended-release layer of EUDRAGIT RL/RS 30D, plasticized with triethyl citrate, and containing the anti-tacking agent talc. A final top coat of Opadry® is then applied. The coated multiparticulates are cured at 60°C for 12 hours, to allow that polymer particles coalesce to form a smooth membrane on extended release beads. A quantity of 251.5 mg of the final beads are filled into hard capsules of size "1".

### Example 3: (AEN) multiparticulates based on pellets prepared by extrusion-spheronization technique

| | mg | % (dry matter) |
|---|---|---|
| AEN tosylate | 52.50 | 30.93 |
| AVICEL RC-591 (Microcrystalline cellulose and carboxymethylcellulose sodium, NF, Ph. Eur.) | 27.30 | 16.08 |
| Lactose powder | 40.20 | 23.68 |
| Water, purified, q.s. | 30.00 | |
| *Sub-total (pellets, non-coated)* | *120.00* | |
| Hydroxypropylcellulose (Klucel®) (Sealcoat) | 2.50 | 1.47 |
| Triacetin | 0.25 | 0.15 |
| Water, purified, q.s. | 20.00 | |
| *Sub-total (sealcoated pellets)* | *122*.*75* | |
| EUDRAGIT® NE 30 D (Polyacrylate Dispersion 30% (Ph. Eur.); Ethyl Acrylate and Methyl Methacrylate Copolymer Dispersion - NF) | 66.70 | 11.79 |
| Hypromellose USP 2910 (e.g.Vivapharm E5) | 2.00 | 1.18 |
| Polysorbate 80 (in form of 33% aq. solution) | 6.00 | 1.18 |
| Talc | 20.00 | 11.78 |
| Water | 35.00 | |
| *Sub-total (solution*/*suspension)* | *129.70* | |
| *Sub-total (sealcoated, extended release pellets)* | *166.76* | |
| Opadry® II (Polyvinyl alcohol based film coating system) (top coat) | 3.00 | 1.77 |
| *Total* | *169.76* | 100.00 |
| Magnesium stearate | 0.24 | |
| Hard gelatine capsule fill mass | 170.0 | |

AEN tosylate, AVICEL RC-591 and lactose powder are mixed and kneaded with purified water. The wet mass is extruded through 0.65 mm. The resulting strains are broken and spheronized in an appropriate spheronizer. The wet spheres are thoroughly dried in a fluid bed dryer. A seal coat of hydroxypropylcellulose, plasticized with triacetin, is applied in a fluid bed equipment. This is followed by an extended-release layer of EUDRAGIT® NE 30 D, hypromellose, plasticized with polysorbate 80, and containing the anti-tacking agent talc. A top coat of Opadry® is finally applied. The coated multiparticulates are cured at 40°C for 24 hours, to allow that polymer particles coalesce to form a smooth membrane on extended release beads. A quantity of 169.76 mg of the final beads are shortly mixed with 0.24 mg magnesium stearate, and the mixture is filled at 170.0 mg into hard capsules of size "2".

### Example 4: (AEN) multiparticulates based on mini-tablets

| *Wet granulation process* | mg | %(dry matter) |
|---|---|---|
| AEN tosylate | 10.50 | 8.75 |
| Lactose hydrous | 65.40 | 54.50 |
| Cornstarch | 36.00 | 30.00 |
| Polyvinylpyrrolidone K-30 | 6.00 | 5.00 |
| Water, purified | q.s. | |
| Talcum | 1.20 | 1.00 |
| Magnesium stearate | 0.90 | 0.75 |
| Total (for 12 mini-tablets) | 120.00 | 100.00 |
| | | |

| *Direct compression process* | mg | % (dry matter) |
|---|---|---|
| AEN tosylate | 10.50 | 8.75 |
| Modified, spray dried lactose (Fast Flo® Lactose) | 103.90 | 86.58 |
| AEROSIL 200 (Colloidal Silicon Dioxide USP, Silica, Colloidal Anhydrous Ph. Eur.) | 0.60 | 0.50 |
| Stearic acid | 2.50 | 2.08 |
| Magnesium stearate | 2.50 | 2.08 |
| Total (for 12 mini-tablet kernels) | 120.00 | 100.00 |

A first option is to prepare the tablets by a wet granulation process. AEN tosylate, lactose hydrous, cornstarch and polyvinylpyrrolidone K-30 are mixed. The mixture is wet granulated with purified water, dried and calibrated. The dry granules are mixed with talcum and magnesium stearate and compressed to double-radius, biconvex kernels of 10 mg, with a diameter of 2.0 mm.

As an alternative, the tablets are prepared by a direct compression process. AEN tosylate and lactose are intimately mixed, Part of the mixture is shortly pre-mixed with AEROSIL, stearic acid and magnesium stearate. The two mixtures are shortly mixed and compressed to tablet kernels as above.

| *Film Coating* | mg | % (dry matter) |
|---|---|---|
| Tablet kernels | 120.00 | 82.76 |
| Opadry® II Clear (seal coat) | 6.00 | 4.14 |
| Aqueous ethyl cellulose dispersion (Surelease) (25% dispersion) | 60.80 | 10.48 |
| Opadry® II Clear (pore former) | 3.80 | 2.62 |
| Water, purified | q.s. | |
| Total | 145.00 | 100.00 |

To prevent any interaction with the drug substance, the kernels are coated with an Opadry seal coat in a side-vented pan. The extended-release coating of Surelease is mixed with purified water and Opadry II Clear as pore former. The coating solution/suspension is again applied in a side-vented pan. The final tablets are cured for 24 hours at 40°C to allow coalescence of the latex particles. Twelve coated mini-tablets are filled into a hard capsule to provide the final product.

### Example 5: Fixed-dose combination of AEN as multiparticulates with valsartan, filled into hard capsules

Valsartan may be used over a dose range of 80 mg to 320 mg daily, administered once a day. AEN multiparticulates of a dose of 6 mg are shown in Example 3. Valsartan multiparticulates are prepared as immediate-release, high-concentrated pellets by extrusion/spheronization as follows.

| | mg | % (dry matter) |
|---|---|---|
| Valsartan | 80.00 | 65.04 |
| AVICEL PH-101 | 30.00 | 24.39 |
| Lactose powder | 9.00 | 7.32 |
| Polyvinyl pyrrolidone K-30 | 1.00 | 0.81 |
| Water, purified, q.s. | q.s | |
| *Sub-total (pellets, non-coated)* | *120.00* | 97.56 |
| Hydroxypropylcellulose (Klucel®) (Sealcoat) | 2.50 | 2.03 |
| Polyethylene glycol 6000 | 0.50 | 0.41 |
| Water, purified, q.s. | q.s. | |
| Total (sealcoated pellets) | 123.00 | 100.00 |
| | | |

| *Fixed dose combination product* | mg | |
|---|---|---|
| AEN extended release pellets (Ex. 3) | 169.76 | |
| Valsartan pellets | 123.00 | |
| Magnesium stearate | 0.24 | |
| Total | 293.0 | |

Valsartan, AVICEL PH-101, lactose powder and polyvinyl pyrrolidone are mixed and kneaded with purified water. The wet mass is extruded through 0.65 mm. The resulting strains are broken and spheronized in an appropriate spheronizer. The wet spheres are thoroughly dried in a fluid bed dryer. A seal coat of hydroxypropylcellulose, plasticized with polyethylene glycol, is applied in a fluid bed equipment. A quantity of 169.76 mg of the AEN pellets of Example 3, 123.0 mg of valsartan pellets and 0.24 mg of magnesium stearate are shortly mixed, and the mixture is filled at 170.0 mg into hard capsules of size "0".

### Example 6: Fixed-dose combination of (AEN) as multiparticulates based on microcrystalline cellulose pellets, with cilostazol, compressed to layer tablets

The recommended dosage of cilostazol is 100 mg BID. A dose of 50 mg BID should be considered during co-administration of certain inhibitors of CYP3A4 and CYP2C19. The AEN extended release formulation used for the fixed-dose combination product is described in Example 3. A dose of 5.0 mg AEN corresponds to 42.44 mg of pellets according to Example 3.

| | mg | % (dry matter) |
|---|---|---|
| AEN tosylate pellets (according to Ex. 3) | 42.44 | 19.29 |
| Cilostazol (microfine powder) | 50.00 | 22.73 |
| AVICEL PH-101 | 75.00 | 34.09 |
| Modified, spray dried lactose (Fast Flo® Lactose) | 27.26 | 12.39 |
| Crospovidone (Kollidone CL) | 22.00 | 10.00 |
| Talc | 2.20 | 1.00 |
| Magnesium stearate | 1.10 | 0.50 |
| Total | 220.00 | 100.00 |

Cilostazol, AVICEL PH-101, modified, spray dried lactose and crospovidone are intimately mixed. Part of this mixture is shortly pre-mixed with talc and magnesium stearate. The residual part is blended with AEN tosylate pellets according to Ex, 3. The pre-mix with talc and magnesium stearate, and the residual mix with the pellets are shortly blended. The final mixture is compressed on a tableting equipment using a pre-compression station to cylindrical, biconvex tablets.

### Example 7: (AEN) single-unit matrix tablet based on hydrophilic polymers

The following example uses a technology in analogy to US 5,135,757 (TimeRx®, Penwest).

| | mg | % (dry matter) |
|---|---|---|
| AEN tosylate | 65.61 | 24.30 |
| Xanthan gum | 60.10 | 22.26 |
| Locust bean gum | 40.07 | 14.84 |
| Lactose, hydrous | 100.17 | 37.10 |
| Water, purified | q.s | |
| Talc | 2.70 | 1.00 |
| Magnesium stearate | 1.35 | 0.50 |
| *Sub-Total* | *270.00* | 100.00 |
| Optional taste-masking coat (e.g. based on Opadry® taste mask film coating system, Colorcon) | 10.00 | |
| Total | 280.00 | |

Xanthan gum, locust bean gum and lactose are intimately blended within a high shear mixer. Purified water is added, and the mass is kneaded until a sharp rise in power consumption occurs. Thereafter the mass is calibrated, dried in a fluid bed dryer and passed through a 20 mesh screen. The dried granules are intimately mixed in a V-cone blender with AEN tosylate. A small part of the mixture is shortly blended with talc and magnesium stearate. Thereafter this pre-blend and the residual mass are shortly blended to provide the ready-to-compress mass. The mass is compressed to oblonge, biconvex tablets with breaking score, which may be film-coated by any appropriate taste-masking film coat.

### Example 8: Osmotically controlled oral delivery system comprising (AEN) ("Push-Pull")

| | mg | % (dry matter) | Function |
|---|---|---|---|
| **Drug layer** | | | |
| AEN tosylate | 26.24 | 21.87 | Drug substance |
| Polyethylene oxide NF (Polyox WSR N-80) (200 kDa) | 68.16 | 56.88 | Viscous polymer |
| Sodium chloride | 20.00 | 16.67 | Osmotic agent |
| Povidone K-30 | 5.00 | 4.17 | Binder |
| Water, purified | q.s. | | |
| Butylated hydroxytoluene NF | 0.10 | 0.10 | Antioxydant |
| Magnesium stearate | 0.50 | 0.42 | Lubricant |
| *Sub-Total* | *120.00* | 100.00 | |

| **Push layer** | | | |
|---|---|---|---|
| Polyethylene oxide NF (Polyox WSR-303) (7,000 kDa) | 63.60 | 63.60 | Swelling polymer |
| Sodium chloride | 30.00 | 30.00 | Osmotic agent |
| Hypromellose type 2910 (Pharmacoat 606) | 5.00 | 5.00 | Dye |
| Iron oxide | 1.00 | 1.00 | Binder |
| Magnesium stearate | 0.30 | 0.30 | Lubricant |
| Butylated hydroxytoluene NF | 0.10 | 0.10 | Antioxydant |
| *Sub-Total* | *100.00* | 100.00 | |

| **Semipermeable membrane** | | | |
|---|---|---|---|
| Cellulose acetate (Eastman CA-398-10 NF/EP) | 19.00 | 95.00 | Coating agent |
| Macrogol 3350 (PEG 3350 kDa) | 1.00 | 5.00 | Plasticizer/pore former |
| Acetone (95 wt-%) | q.s. | | |
| Water, purified (5 wt-%) | q.s. | | |
| *Sub-Total* | *20.00* | 100.00 | |
| Total | 240.00 | | |

### Drug layer

AEN tosylate, polyethylene oxide and sodium chloride were mixed and granulated by an aqueous solution of povidone in a fluid-bed equipment. The granulated, dried mass was passed through a screen. Part of the mass was shortly pre-mixed with butylated hydroxytoluene and magnesium stearate, and thereafter the two parts were mixed.

### Push layer

Sodium chloride and iron oxide are mixed and passed through a screen. This mix and polyethylene oxide are granulated with an aqueous solution of hypromellose in a fluid-bed equipment. After drying, the granules are calibrated through an appropriate screen. Part of the granules, butylated hydroxytoluene and magnesium stearate are pre-mixed for a short time, and thereafter blended with the residual mass.

### Layer tablet

On a layer tableting press, the drug layer is compressed at low compression force to a first layer. After feeding the push layer, the tablets are compressed at standard compression force to cylindric, biconvex kernels with a double radius of curvature.

### Semipermeable membrane

In a side-vented pan with appropriate explosion-proof precautions, a solution of cellulose acetate and macrogol in acetone/water is sprayd on the kernels. The coated kernels are thoroughly dried in the equipment.

### Laser drilling

The coated kernels are fed into a laser-drilling equipment and oriented to allow the drug layer to be positioned constantly in the same direction. By a laser pulse of appropriate strength, an exit passageway is drilled through the semipermeable membrane coat to connect the drug layer with the exterior of the system. A final drying process follows to remove any residual solvent from the coating process.

### Example 9: Fixed-dose combination of AEN with valsartan as compression coated tablet

A single-unit AEN hydrophilic matrix tablet is compression coated with valsartan immediate release.

| | mg | % (dry matter) |
|---|---|---|
| Hydrophilic matrix tablet according to Ex. 7 (40% mass only, corresponding to 10 mg AEN) | 108.00 | |
| Valsartan | 320.00 | 64.00 |
| Lactose, hydrous | 100.00 | 20.00 |
| Corn starch | 46.20 | 9.24 |
| Povidone K-90 | 6.00 | 1.20 |
| Water, purified | q.s. | |
| Corn starch | 19.80 | 3.96 |
| Sodium starch glycolate (type A) (PRIMOJEL®) | 3.00 | 0.60 |
| Magnesium stearate | 5.00 | 1.00 |
| *Sub-Total (Press Coat)* | *500.00* | 100.00 |
| *Total* | 608.00 | |

Valsartan, lactose, corn starch and povidone K-90 are pre-mixed in a high shear mixer and thereafter kneaded with purified water. The wet mass is passed through a rotating sieve with knifes and dried in a fluid bed. The dry granules are calibrated to break down larger agglomerates. Corn starch, sodium starch glycolate (type A) and magnesium stearate are shortly pre-mixed. The two parts are combined and shortly mixed, to provide the final mass for tableting. On a tablet press equipped with three filling stations, on station "1" a first part of the valsartan mix is dosed and pre-compressed at low pressure to a soft layer. At station "2", the AEN tosylate hydrophilic matrix tablet is fed onto the layer prepared on station "1". Finally, on station "3" the residual amount of the valsartan mix is dosed, and the final, biconvex, cylindrical, press-coated tablet is compressed at normal tableting pressure.

### Example 10: Transdermal reservoir type patch for (AEN)

### Reservoir Gel:

A reservoir gel comprising 26 wt.% AEN, 2 wt.% hydroxypropyl cellulose, and 95 wt.% alcohol USP [ethanol 96% EP] is prepared by dissolving AEN in alcohol, and adding hydroxypropyl cellulose with mixing. The AEN gel loading is 21 mg/cm².

### Adhesive Composition:

An adhesive composition is made by mixing high molecular weight polyisobutylene (PIB) (MW 1,200 kD), low molecular weight polyisobutylene (MW 35 kD) and light mineral oil in a weight ratio of 1 : 1.25 :2 . A layer of 50 µm thickness of the PIB adhesive is cast onto a film of 75 µm thickness of siliconized polyethylene terephthalate release liner. The adhesive side of the resulting two layer sub-assembly is laminated to a 50 µm film of ethylene-vinyl acetate (EVA) (9% VA).

### Final delivery system:

The gelled AEN-ethanol mixture is placed on the EVA membrane. A backing membrane comprised of aluminized polyethylene terephthalate with an EVA heat sealable coating is laid over the gels and heat-sealed to the EVA copolymer using a rotary heat seal machine. Finished systems are punched from laminate using a circular punch and placed in sealed pouches to prevent loss of volatile components.

### Example 11: Transdermal matrix type patch for (AEN)

### Preparation of dried adhesive:

Dried adhesive is prepared by coating a solution of a specific adhesive copolymer at a thickness of 500 µm onto a release liner. The coated release liner is oven dried to remove solvent and reduce the amount of residual monomers. The dried adhesive is stripped off the release liner and stored in a container.

### Preparation of polypropylene backing:

A spun-bonded polypropylene nonwoven web (available as LUTRASIL® LS-4460) is treated in a multi-step procedure to produce a backing layer.

### Preparation of a AEN tosylate transdermal delivery device:

A solution of vinylpyrrolidone-vinyl acetate copolymer (Kollidon VA 64) in isopropanol, and adhesive (dried 90/10 isooctyl acrylate/acrylic acid copolymer) in a mixture of ethyl acetate and isopropanol are blended to afford a homogeneous coating formulation. The formulation is coated through an extrusion die onto a release liner (3M Scotchpak™ 1022 Release Liner). The coated release liner is oven dried. The polypropylene backing prepared above is laminated to the coated liner. The laminate is die cut into patches.

### Example 12: 2-Aminoethylnitrate (AEN) tosylate pellet formulations for sustained release based on organic ethylcellulose coating

| Formulation No. | 1 | 2 | 3 | 4 | 5 |
|---|---|---|---|---|---|
| | % | % | % | % | % |
| AEN tosylate | 32.62 | 30.51 | 28.65 | 1.53 | 1.53 |
| Sugar Starch pellets (0,8 - 1,00 mm) | 45.81 | 42.85 | 40.24 | 76.66 | 76.66 |
| Povidone K25 | 0.82 | 0.76 | 0.72 | 0.08 | 0.08 |
| Ethylcellulose | 9.52 | 11.87 | 13.94 | 9.97 | 9.97 |
| Talc | 9.52 | 11.87 | 13.94 | 9.97 | 9.97 |
| Triethyl citrate | 1.71 | 2.14 | 2.51 | 1.79 | 1.79 |

AEN tosylate is dissolved in purified water containing dissolved Povidone K25 (approx. 25.9% solids). Sugar starch pellets of appropriate size are layered with the AEN tosylate-solution in a fluid bed equipment, and the layered pellets are dried in the fluid bed at a temperature of approx. 46°C. In a second step an organic solution of ethylcellulose (type N10 in isopropanol, supplier: Shin-Etsu) containing talc as anti-sticking agent and triethyl citrate as plasticiser are sprayed onto the API pellets in the fluid bed system. Finally, the coated pellets are dried in the fluid bed system at 36°C for approx. 5 min.

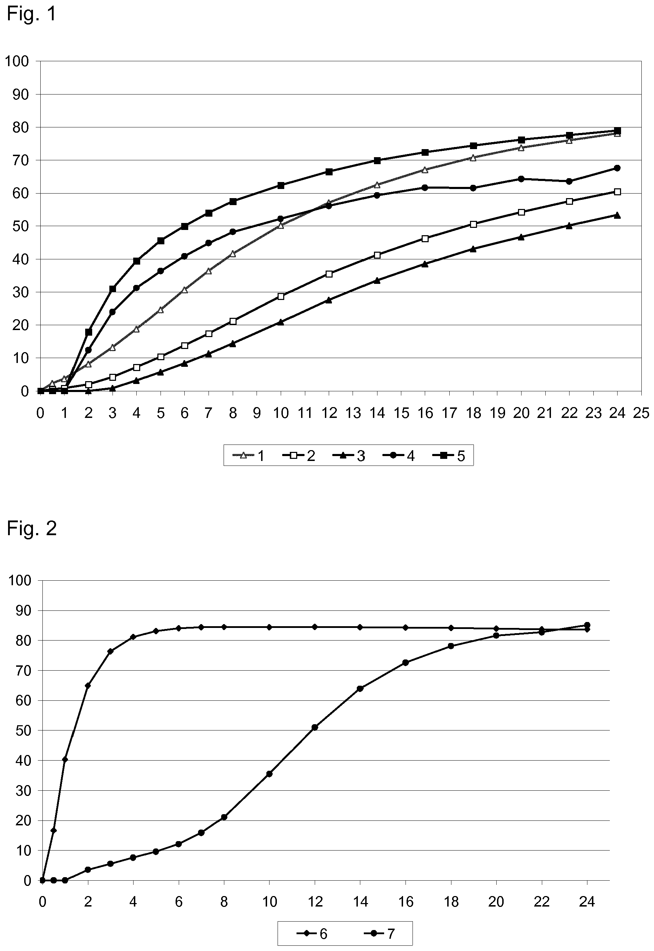

Results of dissolution studies (Ph. Eur.; paddle, pH 6.8, 37°C) are shown in Figure 1.

### Example 13: (AEN) multiparticulates based on sugar starch pellets prepared by layering technique with aqueous ethylcellulose and a double layer of aqueous ethylcellulose and Kollicoat SR

| Formulation No. | 6 | 7 |
|---|---|---|
| | % | % |
| AEN tosylate | 32.97 | 1.4 |
| Sugar Starch pellets (0.85 - 1.00 mm) | 46.31 | 69.0 |
| Povidone K25 | 0.82 | - |
| Ethylcellulose | 16.04 | 14.1 |
| Kollicoat SR | - | 8.4 |
| Triethyl citrate | 3.86 | 4.2 |
| Talc | - | 3.0 |

Formulation No. 6: AEN tosylate is dissolved in purified water containing dissolved Povidone (approx. 25.5% solids). Sugar starch pellets of appropriate size are layered with the AEN tosylate-solution in a fluid bed equipment, and the layered pellets are dried in the fluid bed at a temperature of approx. 46 °C. In a second step an aqueous dispersion of ethylcellulose (supplier: FMC BioPolymer) containing triethyl citrate as plasticiser is sprayed onto the API pellets in the fluid bed system. Finally, the coated pellets are dried in the fluid bed system at 34°C for approx. 15 min.

Formulation No. 7: AEN tosylate is dissolved in purified water (approx. 25.5% solids). Sugar starch pellets of appropriate size are layered with the AEN tosylate-solution in a fluid bed equipment, and the layered pellets are dried in the fluid bed at a temperature of approx. 46 °C. As retard coating an aqueous dispersion of ethylcellulose (supplier: FMC BioPolymer) containing triethyl citrate as plasticiser is sprayed onto the API pellets in the fluid bed system. In a second step an aqueous dispersion of Kollicoat SR (supplier: BASF) is layered onto the pellets. Finally, the coated pellets are dried in the fluid bed system at 34°C for approx. 15 min.

Results of dissolution studies (Ph. Eur.; paddle, pH 6.8, 37°C) are shown in Figure 2.

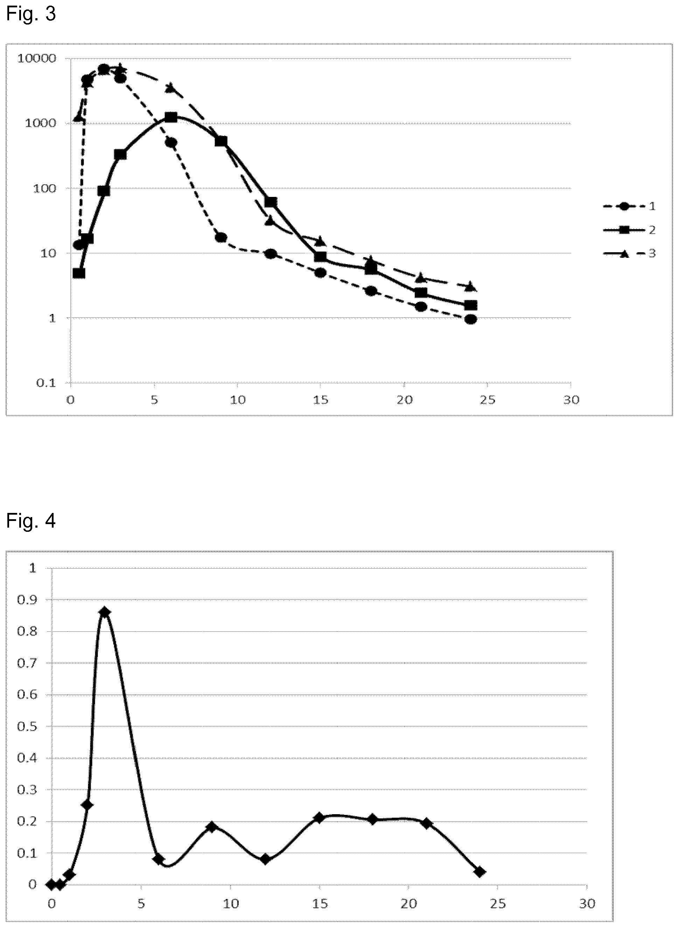

When the AEN tosylate layered sugar starch pellets are coated with an aqueous ethylcellulose layer, the release is, quite independent from the loading of the pellets with API, limited to not more than approx. 3 hours for a complete release under the reported standard conditions of the release test. However, *in vivo* data obtained in dogs (see Figure 3) showed clearly that with this formulation (formulation 6), surprisingly and unexpectedly, already a sustained release effect of approx. 24 h could be achieved. However, the sustained release effect of formulation 6 drops to lower amounts of AEN tosylate in the plasma after 12 hours.

In order to further evaluate these sustained release systems containing AEN tosylate also the final coating using organic solutions of ethylcellulose have been evaluated (formulation 7). These systems have the advantage that the coating layer is smoother and more compact compared to the aqueous systems normally leading to an enhanced sustained release effect. However, surprisingly, the amount of AEN tosylate in the pellets in these systems (Figure 1) had an important effect on the dissolution profiles, but all formulations showed an enhanced sustained release effect as expected. The dissolution profiles were stopped after 24 hours, although they should show release of 36 hours or more.

By using this organic coating system the dissolution can be shifted to longer release times giving the possibility to trigger the *in vivo* release profiles to suitable long lasting releasing formulations dependent on the load of AEN tosylate and the amount of ethylcellulose.

### Example 14: Effects of (AEN) tosylate, valsartan and (AEN) tosylate/valsartan combinations in a rat model for pharmacological activity using blood pressure as a marker for activity

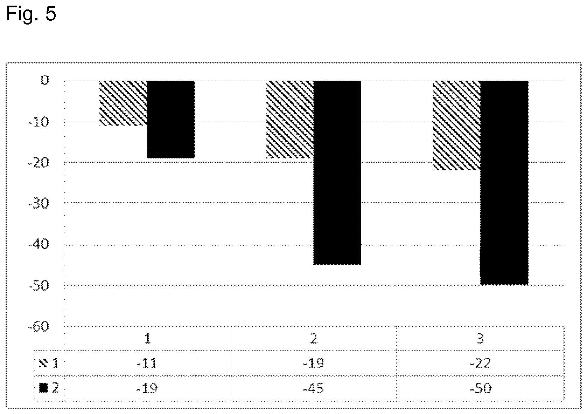

SHR rats were treated with L-NAME to increase blood pressure. Following treatment with L-NAME rats were treated continuously for two weeks with either AEN tosylate (1) with a continuously infused dose (using an osmotic pump) of 0.5 mg/kg/h (column 1 in Fig. 5), valsartan at 5 mg/kg, single daily dose (column 2 in Fig. 5) or AEN tosylate plus valsartan (combined doses, group 3 in Fig. 5). Effects on blood pressure were measured after 1 week and after 2 weeks of treatment.

As expected valsartan lowered blood pressure in test animals (column 2 in Figure 5). Surprisingly AEN tosylate showed continued pharmacological activity without showing signs of NO-tolerance induction (column 1 in Fig. 5). The combination of the two treatments led to an additive effect of the pharmacological activity, again without induction of NO-tolerance.

## Claims

1. An extended release composition of an amino-C₂-C₆-alkyl nitrate and of pharmaceutically acceptable salts thereof, wherein release is over 4-24 hours.

2. An extended release composition according to claim 1, wherein the amino-C₂-C₆-alkyl nitrate is selected from 4-aminobutyl nitrate, 3-aminopropyl nitrate, 2-amino-1-methylethyl nitrate, and 2-aminoethyl nitrate.

3. An extended release composition according to claim 1, wherein the amino-C₂-C₆-alkyl nitrate is 2-aminoethyl nitrate.

4. An extended release composition according to anyone of claims 1 to 3, wherein the pharmaceutically acceptable salt is the tosylate.

5. An extended release composition according to anyone of claims 1 to 4 further comprising another pharmaceutically active compound in a fixed-dose combination.

6. An extended release composition according to claim 5 wherein the other pharmaceutically active compound is selected from the group of angiotensin receptor blockers, angiotensin enzyme inhibitors, renin antagonists, beta blockers, calcium channel blockers, endothelin antagonists, statins, biguanides, glitazones, sulfonureas, SGLT2 antagonists, DPP-4 inhibitors, antithrombotics, antiplatelets, anticoagulants, antianginal drugs, vasodilators, and aldosterone antagonists.

7. An extended release composition according to claim 5 wherein the other pharmaceutically active compound is selected from valsartan, azilsartan, mazisentan, cilostazol, pioglitazone, sitagliptin and linagliptin.

8. An extended release composition according to anyone of claims 1 to 7 as an oral dosage form, wherein the active ingredient or ingredients are embedded in a non-ionic polymer matrix and optionally coated.

9. An extended release composition according to claim 8 in the form of multiparticulates.

10. An extended release composition according to claim 8 in the form of multiparticulates filled into hard capsules.

11. An extended release composition according to claim 8 in the form of multiparticulates compressed to a tablet.

12. An extended release composition according to claim 8 in the form of an osmotic drug delivery system.

13. An extended release composition according to anyone of claims 5 to 7 as a transdermal patch.

14. An extended release composition according to claim 13 in the form of a drug-in-adhesive patch.

15. An extended release composition according to claim 13 in the form of a reservoir patch.

16. A therapeutically effective amount of an extended release composition of 2-aminoethyl nitrate for use in a method of treatment of arterial hypertension, Raynaud's disease, morbus Menière, or argininosuccinic aciduria (ASA).

## Patentansprüche

1. Zusammensetzung mit verlängerter Freisetzung eines Amino-C₂-C₆-alkylnitrats und pharmazeutisch annehmbarer Salze davon, wobei die Freisetzung über 4 bis 24 Stunden erfolgt.

2. Zusammensetzung mit verlängerter Freisetzung nach Anspruch 1, wobei das Amino-C₂-C₆-alkylnitrat aus 4-Aminobutylnitrat, 3-Aminopropylnitrat, 2-Amino-1-methylethylnitrat und 2-Aminoethylnitrat ausgewählt ist.

3. Zusammensetzung mit verlängerter Freisetzung nach Anspruch 1, wobei es sich bei dem Amino-C₂-C₆-alkylnitrat um 2-Aminoethylnitrat handelt.

4. Zusammensetzung mit verlängerter Freisetzung nach einem der Ansprüche 1 bis 3, wobei es sich bei dem pharmazeutisch annehmbaren Salz um das Tosylat handelt.

5. Zusammensetzung mit verlängerter Freisetzung nach einem der Ansprüche 1 bis 4, ferner umfassend eine weitere pharmazeutisch aktive Verbindung in einer Fixdosiskombination.

6. Zusammensetzung mit verlängerter Freisetzung nach Anspruch 5, wobei die andere pharmazeutisch aktive Verbindung aus der Gruppe der Angiotensinrezeptorblocker, Angiotensinenzymhemmer, Reninantagonisten, Betablocker, Calciumkanalblocker, Endothelinantagonisten, Statine, Biguanide, Glitazone, Sulfonharnstoffe, SGLT2-Antagonisten, DPP-4-Hemmer, Antithrombotika, Thrombozytenhemmer, Antikoagulanzien, antianginösen Medikamente, Vasodilatatoren und Aldosteronantagonisten ausgewählt ist.

7. Zusammensetzung mit verlängerter Freisetzung nach Anspruch 5, wobei die andere pharmazeutisch aktive Verbindung aus Valsartan, Azilsartan, Mazisentan, Cilostazol, Pioglitazon, Sitagliptin und Linagliptin ausgewählt ist.

8. Zusammensetzung mit verlängerter Freisetzung nach einem der Ansprüche 1 bis 7 als orale Dosierungsform, wobei der Wirkstoff oder die Bestandteile in einer nichtionischen Polymermatrix eingebettet und gegebenenfalls beschichtet sind.

9. Zusammensetzung mit verlängerter Freisetzung nach Anspruch 8 in Form von Multipartikeln.

10. Zusammensetzung mit verlängerter Freisetzung nach Anspruch 8 in der Form von in Hartkapseln gefüllten Multipartikeln.

11. Zusammensetzung mit verlängerter Freisetzung nach Anspruch 8 in Form von Multipartikeln, die zu einer Tablette gepresst wurden.

12. Zusammensetzung mit verlängerter Freisetzung nach Anspruch 8 in Form eines osmotischen Wirkstoffabgabesystems.

13. Zusammensetzung mit verlängerter Freisetzung nach einem der Ansprüche 5 bis 7 als transdermales Pflaster.

14. Zusammensetzung mit verlängerter Freisetzung nach Anspruch 13 in Form eines Matrixpflasters ("drug-in-adhesive"-Pflaster).

15. Zusammensetzung mit verlängerter Freisetzung nach Anspruch 13 in Form eines Reservoirpflasters.

16. Therapeutisch wirksame Menge einer Zusammensetzung mit verlängerter Freisetzung aus 2-Aminoethylnitrat zur Verwendung in einem Verfahren zur Behandlung von arterieller Hypertonie, der Raynaud-Krankheit, der Meniere-Krankheit oder von Argininosuccino-Azidurie (ASA).

## Revendications

1. Composition à libération prolongée d'un nitrate d'amino-(alkyle en C₂-C₆) et de sels pharmaceutiquement acceptables de celui-ci, dans laquelle la libération se produit sur 4 à 24 heures.

2. Composition à libération prolongée selon la revendication 1, dans laquelle le nitrate d'amino-(alkyle en C₂-C₆) est choisi parmi le nitrate de 4-aminobutyle, le nitrate de 3-aminopropyle, le nitrate de 2-amino-1-méthyléthyle et le nitrate de 2-aminoéthyle.

3. Composition à libération prolongée selon la revendication 1, dans laquelle le nitrate d'amino-(alkyle en C₂-C₆) est le nitrate de 2-aminoéthyle.

4. Composition à libération prolongée selon l'une quelconque des revendications 1 à 3, dans laquelle le sel pharmaceutiquement acceptable est le tosylate.

5. Composition à libération prolongée selon l'une quelconque des revendications 1 à 4 comprenant en outre un autre composé pharmaceutiquement actif dans une combinaison à dose fixe.

6. Composition à libération prolongée selon la revendication 5 dans laquelle l'autre composé pharmaceutiquement actif est choisi dans le groupe des antagonistes de récepteur d'angiotensine, inhibiteurs d'enzyme angiotensine, antagonistes de rénine, bêtabloquants, inhibiteurs calciques, antagonistes d'endothéline, statines, biguanides, glitazones, sulfonurées, antagonistes de SGLT2, inhibiteurs de DPP-4, antithrombotiques, agents antiplaquettaires, anticoagulants, médicaments antiangineux, vasodilatateurs et antagonistes d'aldostérone.

7. Composition à libération prolongée selon la revendication 5 dans laquelle l'autre composé pharmaceutiquement actif est choisi parmi le valsartan, l'azilsartan, le mazisentan, le cilostazol, la pioglitazone, la sitagliptine et la linagliptine.

8. Composition à libération prolongée selon l'une quelconque des revendications 1 à 7 sous la forme d'une forme pharmaceutique orale, dans laquelle la substance ou les composants actifs sont incorporés dans une matrice polymère non ionique et facultativement enrobés.

9. Composition à libération prolongée selon la revendication 8 sous la forme de multiparticules.

10. Composition à libération prolongée selon la revendication 8 sous la forme de multiparticules remplies dans des capsules dures.

11. Composition à libération prolongée selon la revendication 8 sous la forme de multiparticules pressées sous la forme d'un comprimé.

12. Composition à libération prolongée selon la revendication 8 sous la forme d'un système d'administration de médicament osmotique.

13. Composition à libération prolongée selon l'une quelconque des revendications 5 à 7 sous la forme d'un patch transdermique.

14. Composition à libération prolongée selon la revendication 13 sous la forme d'un patch de médicament dans un adhésif.

15. Composition à libération prolongée selon la revendication 13 sous la forme d'un patch à réservoir.

16. Quantité thérapeutiquement efficace d'une composition à libération prolongée de nitrate de 2-aminoéthyle pour utilisation dans un procédé de traitement de l'hypertension artérielle, la maladie de Raynaud, la maladie de Menière ou l'acidurie argininosuccinique (ASA).
